# EUROPEAN PATENT SPECIFICATION

(11) **EP 3 484 910 B1**
(45) Date of publication and mention of the grant of the patent: **07.06.2023**
(21) Application number: 17737821.3
(22) Date of filing: 12.07.2017
(51) Int. Cl.: C07K 14/505, A61K 38/00

(54) **GLYCOPROTEIN WITH REDUCED ACETYLATION RATE OF SIALIC ACID RESIDUES**
GLYCOPROTEIN MIT REDUZIERTER ACETYLIERUNGSRATE VON SIALSÄURERÜCKSTÄNDEN
GLYCOPROTÉINE À TAUX RÉDUIT D'ACÉTYLATION DE RÉSIDUS D'ACIDE SIALIQUE

(30) Priority: 12.07.2016 EP 16179065
(43) Date of publication of application: 22.05.2019
(73) Proprietor: Hexal AG, 83607 Holzkirchen (DE)
(72) Inventor: KRONTHALER, Ulrich, 83607 Holzkirchen (DE); TORELLA, Claudia, 83607 Holzkirchen (DE)
(74) Representative: Michalski Hüttermann & Partner Patentanwälte mbB
(86) International application number: PCT/EP2017/067615
(87) International publication number: WO 2018/011302

(56) References cited:
- WO-A1-03/038100
- US-A1- 2012 045 816
- US-A1- 2014 288 281
- SHAHROKH ZAHRA ET AL: "Erythropoietin Produced in a Human Cell Line (Dynepo) Has Significant Differences in Glycosylation Compared with Erythropoietins Produced in CHO Cell Lines", MOLECULAR PHARMACEUTICS, vol. 8, no. 1, January 2011 (2011-01), pages 286-296, XP002761427, ISSN: 1543-8384
- HUA SERENUS ET AL: "Technologies for glycomic characterization of biopharmaceutical erythropoietins", TRAC, TRENDS IN ANALYTICAL CHEMISTRY, ELSEVIER, AMSTERDAM, NL, vol. 68, 25 March 2015 (2015-03-25), pages 18-27, XP029575015, ISSN: 0165-9936, DOI: 10.1016/J.TRAC.2015.02.004
- CHRISTIAN REICHEL AND GÜNTER GMEINER: "Erythropoietin and Analogs", 1 January 2009 (2009-01-01), HANDBOOK OF EXPERIMENTAL PHARMACOLOGY/ DOPING IN SPORTS: BIOCHEMICAL PRINCIPLES, EFFECTS AND ANALYSIS, SPRINGER VERLAG, DE, PAGE(S) 251 - 254, XP008122894, ISBN: 978-3-540-79087-7 page 259 page 277 - page 280
- REICHEL CHRISTIAN: "Differences in sialic acid O-acetylation between human urinary and recombinant erythropoietins: a possible mass spectrometric marker for doping control.", DRUG TESTING AND ANALYSIS 2013 NOV-DEC, vol. 5, no. 11-12, November 2013 (2013-11) , pages 877-889, XP002761428, ISSN: 1942-7611
- KIM MIN-SUNG ET AL: "Enzymatic deglycosylation of glycoproteins.", METHODS IN ENZYMOLOGY 2013, vol. 533, 2013, pages 259-263, XP009191533, ISSN: 1557-7988

## Description

The present invention relates to glycoproteins with reduced acetylation rate of sialic acid residues.

Sialic acid is a generic term for the N- or O-substituted derivatives of neuraminic acid, a monosaccharide with a nine-carbon backbone. After N-acetylneuraminic acid (Neu5Ac), the most frequent species are N-glycolylneuraminic acid (Neu5Gc) and O-acetylated derivatives.

Sialic acids are found widely distributed in animal tissues and to a lesser extent in other organisms, ranging from plants and fungi to yeasts and bacteria, mostly in glycoproteins where they occur at the end of glycans bound to the latter.

The covalent binding of a glycan to a protein represents an evolutionary mechanism by which the diversity of the proteome can be largely increased. The circumstance that multiple, diverse mechanisms evolved for the glycosylation of proteins argues for the evolutionary benefit and overall relevance of this type of protein modification. Such mechanisms range from non-enzymatic glycation to complex post-translational glycosylation, a multi-step and multi-cell compartment process involving several enzymatic modifications.

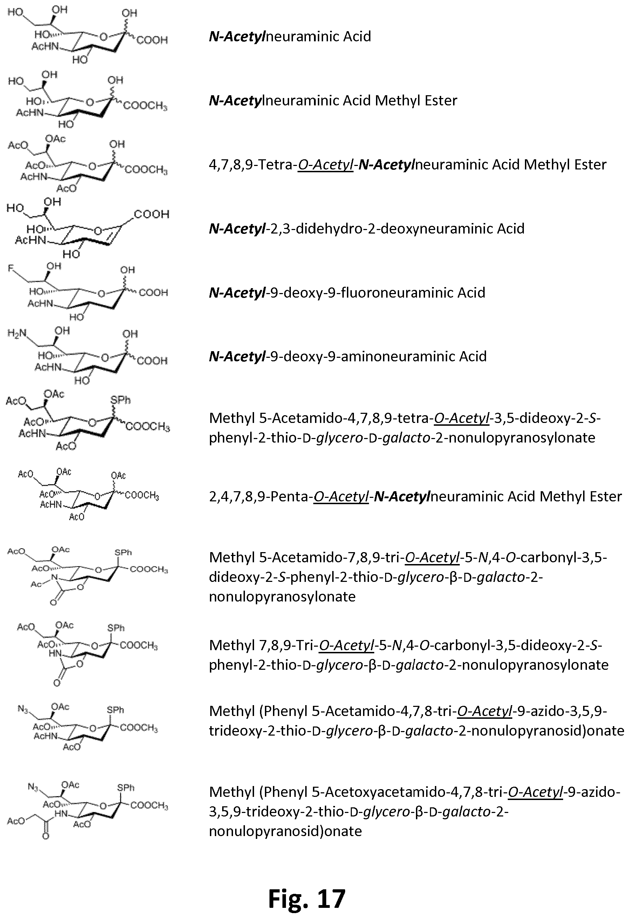

Fig. 17 shows the sialic acid family. One sialic acid molecule may carry one or several acetyl groups. N-Acetyl-neuraminic acid is the most common thereof.

Acetylated sialic acids seem to be involved in many biological phenomena. Glycostructures play a role in protein-protein interactions and they can be a prerequisite for folding into a correct, functional conformation. Their predominance and variability of expression during development and malignancy, also as part of so-called "oncofetal antigens," suggests their participation in numerous physiological and pathological processes. Not surprisingly, genetic defects impairing the synthesis or the attachment of glycan moieties to proteins cause multiple human diseases. These glycostructures can be further modified, thereby introducing additional diversification. One example of such modifications is acetylation. Its role as a potent regulator of cellular interactions classifies acetylation with other biochemical regulations of cell function such as protein phosphorylation, or modification with N-acetylglucosamine (GlcNAc) and methylation.

Acetylation of sialic acid obviously further increases diversity of glycosylation, *i.e.* endogenously, the attached sugars are additionally enzymatically modified. Sialic acids are prototypic examples for such modification of terminal glycans, and acetylation is a widespread type of such additional modifications. Naturally occurring sialic acids share a nine-carbon backbone and can be acetylated at all their hydroxyl groups. This means they can be acetylated at positions C4, C7, C8 and C9. Each sialic acid can be modified once, but also multiple derivatives of a single sialic acid are possible, creating several combination patterns, specifically for glycostructures carrying more than one sialic acid.

Considering the clear importance of glycosylation for the function of proteins and the obviously significant space for modifications, substantial efforts went into optimizing the therapeutic properties of proteins by targeted modification of their glycan structures. Recently the first two examples of this new generation of biopharmaceuticals have achieved marketing authorization: mogamulizumab (Poteligeo^{®}) and obinutuzumab (Gazyvaro^{®} / Gazyva^{®}).

However, targeted modification of glycosylation is not limited to monoclonal antibodies. Darbepoetin alfa (Aranesp^{®}) is an example where also the characteristics of a globular protein were improved, in this case through adding two sialylated carbohydrate chains leading to a prolonged efficacy, *i.e*. decreased treatment frequency.

Shahrokh *et al.* (2011) provides a comprehensive comparative analysis of Dynepo and three other commercial erythropoiesis stimulating agents, Eprex, NeoRecormon and Aranesp.

Hua *et al.* (2015) describes technologies for glycomic characterization of biopharmaceutical erythropoietins and discloses an O-deacetylated ESA.

Reichel and Gmeiner (2009) summarize the mode of action of various recombinant ESA products and analytical procedures in the context of EPO doping.

However, while some of these approaches focus on adaptation of serum half-life and other factors, it seems that modification of efficacy in a narrow sense has not yet been in the focus for potential qualitative modifications. This is in particular true for erythropoiesis-stimulating agents (ESA), which are used to increase the gas (oxygen / carbon dioxide) transport capacity of the blood.

One major risk associated with ESA therapy is increased mortality. As most prominent risk, cardiovascular events occur at a high increase of hemoglobin (Hb), which is discussed to be caused by the ESA-evoked increase in red blood cells (RBC).

It is one object of the present invention to provide a new approach for modifying efficacy of therapeutic glycoproteins.

It is another object of the present invention to provide a new approach for modifying erythropoiesis-stimulating agents to increase their efficacy.

It is still another object of the present invention to provide a new approach for modifying erythropoiesis-stimulating agents which have reduced risk of side effects and/or mortality.

These objects are achieved with methods and means according to the independent claims of the present invention. The dependent claims are related to preferred embodiments.

### Summary of the invention

It is to be understood that the terminology used herein is for purposes of describing particular embodiments only, and is not intended to be limiting. It must be noted that, as used in the specification and the appended claims, the singular forms "a," "an", and "the" include singular and/or plural referents unless the context clearly dictates otherwise. It is moreover to be understood that, in case parameter ranges are given which are delimited by numeric values, the ranges are deemed to include these limitation values.

The present invention relates to a method or process of producing an erythropoiesis-stimulating agent (ESA) selected from the group consisting of erythropoietin, a modified erythropoietin or an erythropoietin mimetic, as defined in independent claim 1, with preferred embodiments being defined in dependent claims 2 to 4.

According to one aspect of the invention, a method or process of producing an erythropoiesis-stimulating agent (ESA) selected from the group consisting of erythropoietin, a modified erythropoietin or an erythropoietin mimetic, is provided, wherein the method or process comprises the heterologous expression of said erythropoiesis-stimulating agent (ESA) in a suitable expression system, wherein at least one step or feature is provided that results in a reduced O-acetylation rate of sialic acid residues in said erythropoiesis-stimulating agent (ESA), for the purpose of increasing mean corpuscular hemoglobin (MCH) The method or process comprises the heterologous expression of said ESA in a suitable expression system, wherein at least one step or feature is provided that results in a reduced acetylation rate of sialic acid residues in the ESA.

The erythropoietin receptor (EpoR) is a protein that in humans is encoded by the EPOR gene. EpoR is a 52 kDa peptide with a single carbohydrate chain resulting in an approximately 56-57 kDa protein found on the surface of EPO responding cells. EpoR pre-exists as a dimer which upon binding of a suitable ligand changes its homodimerized state.

The cytoplasmic domains of the EpoR contain a number of phosphotyrosines which, upon the conformational changes caused by ligand binding, are phosphorylated by Jak2, and serve as docking sites for a variety of intracellular pathway activators and Stats (such as Stat5).

The primary role of EpoR is to promote proliferation of erythroid progenitor cells and rescue erythroid progenitors from cell death. Based on current evidence, it is however still unknown whether EpoR directly causes "proliferation and differentiation" of erythroid progenitors *in vivo.*

Another role EpoR is thought to be involved in is to promote erythroid differentiation. EpoR's PI3-K/AKT signaling pathway augments GATA-1 activity. One hypothesis is that erythroid differentiation is primarily dependent on the presence and induction of erythroid transcriptional factors such as GATA-1, FOG-1 and EKLF.

It is also known that EpoR can activate mitogenic signaling pathways and can lead to cell proliferation in erythroleukemic cell lines *in vitro.* EpoR expression can extend as far back as the hematopoietic stem cell compartment. It is, however, unknown whether EpoR signaling plays a permissive or an instructive role in early, multipotent progenitors in order to produce sufficient erythroblast numbers.

The term "acetylation rate of sialic acid residues", as used herein, refers to the overall percentage of sialic acids of a given glycoprotein type which carry one or more acetyl residues. Preferably, the term relates to O-acetyl residues on these sialic acids.

The term "glycoprotein", as used herein, refers to proteins and peptides that contain oligosaccharide chains (glycans) covalently attached to polypeptide side-chains. The carbohydrate is attached to the protein in a cotranslational or posttranslational modification. This process is known as glycosylation.

Preferably, the reduced acetylation is reduced O-acetylation. This applies for all embodiments disclosed herein which recite the term acetylation. In all cases, O-acetylation is a preferred embodiment.

In one embodiment, the step or feature that results in a reduced O-acetylation rate of sialic acid residues is at least one selected from the group consisting of:
a) O-deacetylation of sialic acid residues in glycans of said erythropoiesis-stimulating agent (ESA),
b) reduction of overall glycosylation of said erythropoiesis-stimulating agent (ESA), resulting in a reduction of O-acetylated sialic acid residues,
c) use of an expressor cell line that is capable of expressing erythropoiesis-stimulating agent (ESA) that has de- or non-O-acetylated sialic acid residues, or a reduced O-acetylation rate of sialic acid residues
d) use of an expressor cell line that is capable of expressing erythropoiesis-stimulating agent (ESA) that is deglycosylated, or has reduced glycosylation
e) reduction of sialic acid content in glycans of said erythropoiesis-stimulating agent (ESA), and
f) use of an expressor cell line that is capable of expressing erythropoiesis-stimulating agent (ESA) that has a reduced amount of sialic acid, or lack sialic acids.

The term "expressor cell line", as used herein, relates to a cell line that is capable of expressing the erythropoiesis-stimulating agent (ESA) either homologously or heterologously.

Preferably, steps/features a), b and e) relate to *in vitro* processes that are carried out, *e.g.,* by posttranslational enzymatic or chemical treatment of the glycoprotein.

O-deacetylation of sialic acid residues (step or feature a) can be accomplished, *e.g.,* by posttranslational treatment with a suitable acetylesterase. One example is sialate O-acetylesterase (SIAE), which in humans is encoded by the SIAE gene located on chromosome 11. SIAE catalyzes the removal of O-acetyl ester groups from position 9 of the parent sialic acid.

However, other organisms, and other expressor cell lines, have other types of acetylesterases. Sialate O-acetylesterases are disclosed in Expasy enzyme entry EC 3.1.1.53. These enzyme catalyze, inter alia, the following reaction:

N-acetyl-O-acetylneuraminate + H(2)O <=> N-acetylneuraminate + acetate

It is hence in the routine of the skilled person to silence, delete or mutate the respective acetyltransferase in the respective organisms, and other expressor cell lines on the basis of the teaching disclosed herein.

Reduction of overall glycosylation (step b) can be accomplished, *e.g.,* by posttranslational treatment with suitable enzymes, like NANase II, Endoglycosidase H, *O*-glycosidase, and/or Peptide-N-Glycosidase F (PNGase F). Protocols are described in Kim & Leahy (2013). A chemical approach for the deglycosylation of glycoproteins, which uses trifluoromethanesulfonic acid, is disclosed in Sojar H & Bahl (1987).

Generally, de-glycosylation removes all glycans from a glycoprotein, and hence, all sialic acid residues, or their acetyl substituents, respectively. Deglycosylation thus results, *de facto,* in the removal of acetyl residues conjugated to sialic acids, and thus has similar effects.

Reduction of sialic acid content in glycans of a glycoprotein can be done according to methods disclosed in WO2011061275A1.

Step/feature c) preferably refers to an expressor cell line that (i) exhibits *per se,* a reduced O-acetylation (*i.e.,* in which the acetylation rate has not been artificially manipulated), or (ii) has been modified in such way that O-acetylation of sialic acid residues during post translational protein modification is affected.

This can be accomplished, *e.g.,* by inhibition or reduction of gene expression of a gene coding for an enzyme that catalyzes sialic acid O-acetylation, or expression of a dysfunctional, or inactive enzyme that catalyzes sialic acid O-acetylation, or an enzyme that catalyzes sialic acid O-acetylation with reduced activity.

Step/feature d) preferably refers to an expressor cell line that has for example been modified in such way that protein glycosylation is affected.

This can be accomplished, *e.g.,* by modifying an expressor cell line in such way that it expresses, or overexpresses, heterologous or homologous enzymes that catalyze deglycosylation.

Examples for enzymes that catalyze deglycosylation include NANase II, Endoglycosidase H, O-glycosidase, and/or Peptide-N-Glycosidase F (PNGase F). Overexpression of any of these genes in a suitable expressor cell line, or heterologous expression in a suitable expressor cell line results in deglycosylation during post translational protein modification.

Said heterologous expression can be accomplished *e.g.,* by genetic engineering techniques, including non-transient transfection of a cell with a vector that encodes for a respective enzyme.

Said homologous overexpression can be accomplished *e.g.,* by genetic engineering techniques, including induced overexpression of an intrinsic gene that encodes for a respective enzyme by means of a suitable promoter that has been inserted upstream of the encoding gene.

Said deletion or mutagenesis can be accomplished by methods described elsewhere herein.

As the inventors have shown herein, an erythropoiesis-stimulating agent (ESA) according to the present invention which has a reduced O-acetylation rate of sialic acid residues offers the opportunity to apply a lower dose while achieving the same therapeutic effect. This results in cost savings and decreases the risk of unwanted side effects, such as formation of antibodies, by applying a smaller amount of erythropoiesis-stimulating agent (ESA) to the patient. The possibility to apply lower doses also reduces the volumes to be given to the patient, adding further benefit to such a therapeutic, and increasing patient compliance.

Further, the erythropoiesis-stimulating agent (ESA) according to the present invention which has a reduced O-acetylation rate of sialic acid may reduce a major risk associated with ESA therapy, namely increased mortality. As most prominent risk, cardiovascular events occur at a high increase of Hb, which is discussed to be caused by the ESA-evoked increase in RBC. A therapy with an erythropoiesis-stimulating agent (ESA) according to the present invention leads to an increase of mean corpuscular hemoglobin (MCH) above, while the RBC increase is less pronounced. Taken together, this results in a favourable safety profile, *e.g.* regarding cardiovascular safety and mortality in general.

The erythropoiesis-stimulating agent (ESA) is an erythropoietin, a modified erythropoietin or an erythropoietin mimetic.

The term "erythropoietin", as used herein, comprises the different wild type erythropoietins (Epoetin α, Epoetin β, Epoetin γ, Epoetin δ, Epoetin ε, Epoetin ζ, Epoetin θ, Epoetin κ or Epoetin ω).

The term "modified erythropoietin", as used herein, refers to a protein that relies, structurally and/or sequence-wise, on wild type erythropoietin, but comprises structural modifications in either (i) its amino acid sequence, its (ii) glycosylation pattern or (iii) by addition of other moieties. These modifications do not affect its agonistic interaction with the erythropoietin receptor as such, but either modify said agonistic interaction, or modify other physico-chemical, pharmacological or PK/PD properties thereof, like bioavailability, serum half-life, tissue distribution, efficacy, shelf life, and the like.

The term "erythropoietin mimetic", as used herein, refers to proteins and peptides that agonistically interact with the erythropoietin receptor. Erythropoietin mimetics are disclosed, *inter alia,* in Johnson, & Jolliffe (2000), and US8642545B2. Studies have shown that in the EPO receptor, which is a 484-amino acid glycoprotein with a single transmembrane segment located between extracellular and intracellular domains each of nearly equal size, Phe⁹³ is crucial for binding EPO, as well as binding erythropoietin mimetic peptides (Middleton *et al.* 1999).

Quite obviously, modified erythropoietins and erythropoietin mimetics are two classes that have a large overlap. Hence, some modified erythropoietins can also be considered erythropoietin mimetics and *vice versa.*

In one embodiment of the invention, the erythropoiesis-stimulating agent (ESA) is at least one selected from the group consisting of:
- Epoetin α (Epogen^{®}, ESPO^{®}, Procrit^{®}, Eprex^{®}, Erypo^{®}, Epoxitin^{®}, Globuren^{®}, Epopen^{®}, Epoglobin^{®}, Epox^{®}, Eritrogen^{®})
- Epoetin-β (NeoRecormon^{®}, Epogin^{®})
- Darbepoetin α (Aranesp^{®}, Nespo^{®} )
- CERA (Continuous Erythropoiesis Receptor Activator)
- ErepoXen^{®}
- Albupoetin^{®}
- PT-401
- Epo-Fc
- CEPO (carbamylated EPO)
- MOD-7023
- Epoetin δ (DynEpo^{®})
- GO-EPO
- MK2578

In yet another embodiment of the invention, the step that results in a reduced O-acetylation rate of sialic acid residues also affects bioavailability, exposure, serum half-life or absolute serum concentration of the glycoprotein.

The inventors have for example shown that reduction of sialic acid O-acetylation may on the one hand side reduce bioavailability of an ESA, but at the same time increase MCH, compared to a non-modified ESA, as shown to a degree even overcompensating the lower exposure as measured by the Hb level. Hence, although seemingly contra-productive in terms of Hb achieved, the reduction of bioavailability surprisingly does not translate to a reduced Hb. The example shows that the decreased exposure is even overcompensated by the increased MCH resulting from the treatment with the modified ESA. This may have multiple consequences. From a manufacturing perspective, a lower dose of protein required to correct the patients hemopoiesis may translate into lower costs of goods. From a patient perspective this lower dose together with the decreased exposure due to the fast elimination would imply a further benefit. Exposure to a therapeutic protein correlates with the risk of the individual to develop anti-drug antibodies (ADA). In case of ESAs the likelihood for such ADA formation is typically low. A lower exposure would thus also decrease the immunogenicity risk. Lastly and most importantly, the increase of Hb without the otherwise rather linear increase of RBC decouples the Hb rise from an increased thrombosis risk.

In one embodiment of the invention, said erythropoiesis-stimulating agent (ESA) has at least one selected from the group consisting of:
a) an absolute acetylation rate of ≤ 10 %, and
b) an acetylation rate that is reduced by ≥ 50 %.

As used herein, the term "absolute acetylation rate (%)" refers to the overall percentage of sialic acids of a given erythropoiesis-stimulating agent (ESA) type which carry one or more O-acetylation. It is important to emphasize that in the meaning of the present disclosure, a partial or total deglycosylation would result in a reduction of the acetylation rate, too.

Preferably, the absolute acetylation rate is ≤ 9, 8, 7, 6, 5, 4, 3, 2 or 1 %. Most preferably, the absolute acetylation rate is 0 %. In this embodiment the sialic acids of all glycans of a given glycoprotein are completely de- or non-acetylated.

However, under some conditions the absolute acetylation rate should at the same not be smaller than 0.1; 0.2; 0.3; 0.4 or 0.5 %.

As used herein, the term "acetylation rate that is reduced by X %" refers to relative reduction of sialic acids of a given glycoprotein type which carry one or more acetyl groups compared with (i) a commercially available glycoprotein of identical or similar amino acid sequence, or (ii) on a wildtype glycoprotein of identical or similar amino acid sequence expressed by its natural host, or by a suitable expression system, wherein further in either case, the respective commercially available glycoprotein or the wildtype glycoprotein has not been modified to affect the acetylation rate of sialic acid residues.

Preferably, the acetylation rate is reduced by ≥ 51, 52, 53, 54, 55, 56, 57, 58, 59, 60, 61, 62, 63, 64, 65, 66, 67, 68, 69, 70, 71, 72, 73, 74, 75, 76, 77, 78, 79, 80, 81, 82, 83, 84, 85, 86, 87, 88, 89, 90, 91, 92, 93, 94 or 95 %.

However, under some conditions the acetylation rate should not be reduced by more than 99; 98; 97 or 96 %.

For example, the absolute acetylation rate of an ESA (darbepoietin alpha) according to the invention is < 1 %, while the commercially available ESA of identical or similar amino acid sequence (Aranesp^{®}) has an absolute acetylation rate of 29.5 %. Hence, the acetylation rate is reduced to about 3 % of that of commercially available ESA of identical or similar amino acid sequence.

Preferably, the acetylation rate is determined with at least one method selected from the group consisting of:
- Relative quantitation of sialic acids after cleavage of the glycosidic linkage from the glycoprotein or isolated glycans using an enzymatic (with neuraminidases) or chemical approach (hydrolysis with mild acid)
- Derivatization of sialic acids with 1,2-diamino-4,5-methylenedioxybenzene (DMB) followed by high-performance liquid chromatography with fluorescence detection
- Pertrimethylsilylation followed by GLC (gas-liquid chromatography)-MS
- Thin layer chromatography (radio-TLC or densitometric quantitation)

For an overview of methods suitable for acetylation rate determination, see *e.g.* Reuter and Schauer, 1994). In case the reduction of acetylation rate has been accomplished indirectly, *i.e.,* by partial or total glycosylation, the acetylation rate can as well be determined with, *inter alia,* chemical deglycosylation with trifluoromethane sulfonic acid (disclosed in Sojar & Bahl (1987)).

According to another aspect, the present invention relates to the *in-vitro* use of an erythropoiesis-stimulating agent (ESA), selected from the group consisting of erythropoietin, a modified erythropoietin or an erythropoietin mimetic, comprising a reduced O-acetylation rate of sialic acid residues for the increase of mean corpuscular hemoglobin (MCH).

According to the present application, the use of such erythropoiesis-stimulating agent (ESA) is disclosed for the manufacture of a medicament for the treatment of a human or animal patient or subject.

The application further discloses a pharmaceutical preparation comprising a erythropoiesis-stimulating agent (ESA) according to the above description in a pharmaceutically acceptable carrier.

Because of the increased efficacy, such preparation could be administered in smaller doses or volumes, which in turn may help to decrease drug aggregation risk, increase shelf life, reduce storage needs, reduce administration related discomfort and increase patient compliance.

According to another aspect, the present invention relates to an erythropoiesis-stimulating agent (ESA), selected from the group consisting of erythropoietin, a modified erythropoietin or an erythropoietin mimetic, comprising a reduced O-acetylation rate of sialic acid residues, for use in the treatment of conditions associated with a reduced mean corpuscular hemoglobin (MCH).

In a preferred embodiment of the present invention, said condition is a condition associated with normal red blood cell (RBC) counts but low hemoglobin (Hb).

In a further preferred embodiment of the present invention, said condition is hypochromic anemia.

According to the present application, the human or animal patient or subject can suffer from, is at risk of developing, and/or is diagnosed for, at least one disease or symptom selected from the group consisting of:
- anemia,
- AIDS- and cancer-related diseases and unwanted consequences of related therapies, like chemotherapy, and
- hypoxic syndromes.

Said anemia can have different origins, *e.g.,* kidney diseases deficiency, radiotherapy, cancer such as myelodysplastic syndrome with or without bone marrow suppressive chemotherapy, iron or iron metabolism deficiency, symptomatic anemia in predialysis, or bone marrow deficiency or disease.

According to the present application, the human or animal patient or subject can be subject of a condition or undergoes a treatment selected from the group consisting of:
- hematopoietic stem cell transplantation,
- intensive care,
- need for stimulation of erythropoiesis, pre- or peri-surgery, *e.g.* for autologous blood donation.

The application further discloses a cell for heterologous expression of a glycoprotein according to the above description is provided, which cell
a) is capable of expressing glycoproteins that have de- or non-O-acetylated sialic acid residues, or a reduced O-acetylation rate of sialic acid residues, and/or
b) is capable of expressing glycoproteins that are deglycosylated, or have reduced glycosylation.

The application further discloses the expression of an erythropoiesis-stimulating agent (ESA) that has de- or non-O-acetylated sialic acid residues, or a reduced O-acetylation rate of sialic acid residues, which is accomplished by at least one of:
a) inhibition or reduction of gene expression of a gene coding for an enzyme that catalyzes sialic acid O-acetylation,
b) expression of a dysfunctional, or inactive enzyme that catalyzes sialic acid O-acetylation, or an enzyme that catalyzes sialic acid O-acetylation with reduced activity,
c) inhibition or reduction of the activity of an enzyme that catalyzes sialic acid O-acetylation, and/or
d) heterologous expression or homologous overexpression of a gene coding for an enzymes that catalyzes O-deacetylation of sialic acid residues.

Examples for such enzymes catalyzing sialic acid O-acetylation are shown in the following list, which is however not to be construed as limiting:
- N-acetylneuraminate 7-O(or 9-O)-acetyltransferase (EC 2.3.1.45)
- polysialic-acid O-acetyltransferases (EC 2.3.1.136)
- sialic acid O-acetyltransferase (neuD family)
- α-N-acetyl-neuraminide α-2,8-sialyltransferase 1 (GD3 synthase)
- human sialate-O-acetyltransferase (CasD1)

These enzymes catalyse reactions of *e.g.,* the following types:

Acetyl-CoA + N-acetylneuraminate -> CoA + N-acetyl-7-O(or 9-O)-acetylneuraminate

Acetyl-CoA + an alpha-2,8-linked polymer of sialic acid -> CoA + polysialic acid acetylated at O-7 or O-9

Silencing, deletion or mutagenesis of said gene in a suitable human expressor cell line results in a lacking or dysfunctional enzyme, which in turn leads to a reduced or lacking O-acetylation of sialic acid residues during post-translational protein modification.

However, other organisms, and other expressor cell lines, have other types of sialic acid acetyltransferases. It is hence in the routine of the skilled person to silence, delete or mutate the respective acetyltransferase in the respective organism or expressor cell line on the basis of the teaching disclosed herein.

According to the present application, preferably, said enzyme of option d) catalyzes de-o-acetylation of sialic acid. Examples for such enzymes are shown in the following list, which is however not to be construed as limiting:
- sialate-9-O-acetylesterase (EC 3.1.1.53)
- 9-O-acetyl N-acetylneuraminic acid esterase
- haemagglutinin esterase
- cytosolic sialic acid 9-O-acetylesterase

These enzymes catalyze, inter alia, the following reaction:

N-acetyl-O-acetylneuraminate + H₂O -> N-acetylneuraminate + acetate

Human sialate O-acetylesterase (SIAE) is for example encoded by the SIAE gene located on chromosome 11. SIAE catalyzes the removal of O-acetyl ester groups from position 9 of the parent sialic acid.

Overexpression of a gene from the above list in a suitable human expressor cell line, or heterologous expression in a non-human expressor cell line, results in post-translational O-deacetylation of sialic acid residues during post translational protein modification.

However, other organisms, and other expressor cell lines, have other types of acetylesterases. It is hence in the routine of the skilled person to silence, delete or mutate the respective acetyltransferase in the respective organism or expressor cell line on the basis of the teaching disclosed herein.

The application further discloses that the expression of glycoproteins that are deglycosylated, or have reduced glycosylation, can be accomplished by heterologous expression or homologous overexpression of a gene coding for an enzyme that catalyzes deglycosylation.

The application further discloses that the inhibition or reduction of gene expression can be been achieved by at least one genetical engineering step selected from the group consisting of:
- gene silencing,
- gene knock-down,
- gene knock-out,
- delivery of a dominant negative construct,
- conditional gene knock-out, and/or
- gene alteration with respect to a gene coding for an enzyme that catalyzes sialic acid acetylation or deacetylation of sialic acid residues.

The term "gene expression", as used herein, is meant to encompass at least one step selected from the group consisting of: DNA transcription into mRNA, mRNA processing, non-coding mRNA maturation, mRNA export, translation, protein folding and/or protein transport.

The inhibition or reduction of gene expression of a gene refers to methods which directly interfere with gene expression, encompassing, but not restricted to, inhibition or reduction of DNA transcription, *e.g.,* by use of specific promoter-related repressors, by site specific mutagenesis of a given promoter, by promoter exchange, or inhibition or reduction of translation, *e.g.,* by RNAi induced post-transcriptional gene silencing. The expression of a dysfunctional, or inactive enzyme, or an enzyme with reduced activity, can, for example, be achieved by site specific or random mutagenesis, insertions or deletions within the coding gene.

The inhibition or reduction of the activity of an enzyme can, for example, be achieved by administration of, or incubation with, an inhibitor to the respective enzyme, prior to or simultaneously with protein expression. Examples for such inhibitors include, but are not limited to, an inhibitory peptide, an antibody, an aptamer, a fusion protein or an antibody mimetic against said enzyme, or a ligand or receptor thereof, or an inhibitory peptide or nucleic acid, or a small molecule with similar binding activity. Some inhibitors described in literature for N-acetylneuraminate 7-O(or 9-O)-acetyltransferase activity are iodoacetate, CoA, diethyl carbonate, N-bromosuccinimide, Triton X-100 or p-chloromercuribenzoate.

Other ways to inhibit the enzyme comprise the reduction of specific cofactors of the enzyme in the medium.

Gene silencing, gene knock-down and gene knock-out refer to techniques by which the expression of a gene is reduced, either through genetic modification or by treatment with an oligonucleotide with a sequence complementary to either an mRNA transcript or a gene. If genetic modification of DNA is done, the result is a knock-down or knock-out organism. If the change in gene expression is caused by an oligonucleotide binding to an mRNA or temporarily binding to a gene, this results in a temporary change in gene expression without modification of the chromosomal DNA and is referred to as a transient knock-down.

In a transient knock-down, which is also encompassed by the above term, the binding of this oligonucleotide to the active gene or its transcripts causes decreased expression through blocking of transcription (in the case of gene-binding), degradation of the mRNA transcript (*e.g.* by small interfering RNA (siRNA) or RNase-H dependent antisense) or blocking either mRNA translation, pre-mRNA splicing sites or nuclease cleavage sites used for maturation of other functional RNAs such as miRNA (*e.g.,* by morpholino oligos or other RNase-H independent antisense). Other approaches involve the use of shRNA (small hairpin RNA, which is a sequence of RNA that makes a tight hairpin turn that can be used to silence gene expression via RNA interference), esiRNA (endoribonuclease-prepared siRNAs, which are a mixture of siRNA oligos resulting from cleavage of long double-stranded RNA (dsRNA) with an endoribonuclease), or the activation of the RNA-induced silencing complex (RISC).

Another approach for genetic modifications that can be used in the present context comprises the use of CRISPR Cas (Baumann *et al.,* 2015), TALEN or ZFN (Gaj *et al.,* 2013).

Other approaches to carry out gene silencing, knock-down or knock-out are known to the skilled person from the respective literature, and their application in the context of the present invention is considered as routine.

Gene knock-out refers to techniques by which the expression of a gene is fully blocked, *i.e.* the respective gene is inoperative, or even removed. Methodological approaches to achieve this goal are manifold and known to the skilled person. Examples are the production of a mutant which is dominantly negative for the given gene. Such mutant can be produced by site directed mutagenesis (*e.g.,* deletion, partial deletion, insertion or nucleic acid substitution), by use of suitable transposons, or by other approaches which are known to the skilled person from the respective literature, the application of which in the context of the present invention is thus considered as routine. One example for a newly developed technique which the skilled person would consider as useful in the context of the present invention is knock-out by use of targeted zinc finger nucleases. A respective kit is provided by Sigma Aldrich as "CompoZR knockout ZFN". Another approach encompasses the use of Transcription Activator-Like Effector Nucleases (TALENs).

The delivery of a dominant negative construct involves the introduction of a sequence coding for a dysfunctional enzyme, *e.g.,* by transfection. Said coding sequence is functionally coupled to a strong promoter, in such way that the gene expression of the dysfunctional enzyme overrules the natural expression of the wild type enzyme, which, in turn, leads to an effective physiological defect of the respective enzyme activity.

A conditional gene knock-out allows to block gene expression in a tissue- or time-specific manner. This is done, for example, by introducing short sequences called loxP sites around the gene of interest. Again, other approaches are known to the skilled person from the respective literature, and their application in the context of the present invention is considered as routine.

One other approach is gene alteration which may lead to a dysfunctional gene product or to a gene product with reduced activity. This approach involves the introduction of frame shift mutations, nonsense mutations (*i.e.,* introduction of a premature stop codon) or mutations which lead to an amino acid substitution which renders the whole gene product dysfunctional, or causing a reduced activity. Such gene alteration can for example be produced by mutagenesis (*e.g.,* deletion, partial deletion, insertion or nucleic acid substitution), either unspecific (random) mutagenesis or site directed mutagenesis.

Protocols describing the practical application of gene silencing, gene knock-down, gene knock-out, delivery of a dominant negative construct, conditional gene knock-out, and/or gene alteration are commonly available to the skilled artisan, and are within his routine. The technical teaching provided herein is thus entirely enabled with respect to all conceivable methods leading to an inhibition or reduction of gene expression of a gene coding for an enzyme, or to the expression of a dysfunctional, or inactive enzyme, or an enzyme with reduced activity.

The application further discloses
a) the finding that enzymatic de-O-acetylation of a heterologously expressed agonist to EpoR has a given modified efficacy
b) the disclosure of exemplary enzymes that catalyze O-acetylation or de-O-acetylation in different cellular expression systems
c) the enumeration of technologies available to the skilled person which enable him to generate an expressor cell line that has either an impaired or defect homologous enzyme, or expresses a heterologous enzyme, or overexpresses a homologous enzyme.

Hence, the technical teaching of this application enables the skilled person to develop an expressor cell line that has either impaired or defect O-acetylation or has increased de-O-acetylation, in order to produce an EpoR agonist with a given modified efficacy.

Said cell can be a eukaryotic cell. The term "eukaryotic cell" encompasses, but is not restricted to, animal cells, like, *e.g.,* insect cells, plant cells and fungal cells. Said cell can be an animal cell and/or a plant cell. Said cell can be a mammalian cell.

The application further discloses that the cell can be at least one selected from the group consisting of:
- Baby hamster Kidney cells (*e.g.,* BHK21),
- Chinese hamster ovary cells (*e.g.,* CHO-K1, CHO-DG44, CHO-DXB, or CHO-dhf),
- Mouse myeloma cells (*e.g.,* SP2/0 or NSO),
- Human embryonic kidney cells (*e.g.,* HEK-293),
- Human-retina-derived cells (*e.g.,* PER-C6), and
- Amniocyte cells (*e.g.,* CAP).

The cell can be a recombinant cell. As used herein, the term "recombinant cell" is used to refer to a cell with exogenous and/or heterologous nucleic acid incorporated within, either incorporated stably so as to remain incorporated in clonal expansion of the cells, or introduced transiently into a cell (or a population of cells). Such exogenous and/or heterologous nucleic acid can either code for a heterologous protein to be expressed, or it can affect the inhibition or reduction of gene expression of a gene coding for an enzyme, or the expression of a dysfunctional or inactive enzyme, or an enzyme with reduced activity.

Foregoing description is by way of example only and is not intended as limiting. The invention's scope is defined in the following claims and the equivalents thereto.

### Brief description of the examples and drawings

Additional details, features, characteristics and advantages of the object of the invention are disclosed in the dependent claims, and the following description of the respective figures and examples, which, in an exemplary fashion, show preferred embodiments of the present invention. However, these drawings should by no means be understood as to limit the scope of the invention.

### Figures

Fig. 1: Time course of mean serum levels (n=8-10/group) upon single infusion of human IgG and deglycosylated human IgG in rabbits
Fig. 2: Individual Cₘₐₓ (n=8-10/group) upon single infusion of human IgG and deglycosylated human IgG in rabbits.
Fig. 3: Time course of mean serum levels (n=1 1/group) upon single *s.c.* injection of two batches of the Fc fusion protein Orencia^{®} (abatacept) differing in O-acetylation in rabbits (batch 1 with high, batch 2 with low O-acetylation).
Fig. 4: Individual AUC (n=11/group, bar indicates group mean) upon single *s.c.* injection of two Orencia^{®} batches in rabbits. The term "level of O-acetylation" used herein and in Figs. 5 - 7 and 10 - 13 means "acetylation rate of sialic acid residues", as defined herein elsewhere.
Fig. 5: Relationship between mean AUC and level of O-acetylation (n=11-14/group) upon single *s.c.* injection of two Orencia^{®} batches.
Fig. 6: Individual Cₘₐₓ (n=1 1/group, bar indicates group mean) upon single *s.c.* injection of two Orencia^{®} batches (batch 1 with low and batch 2 with high O-acetlyation) in rabbits.
Fig. 7: Relationship between mean Cₘₐₓ and level of O-acetylation (n=11/group) upon single *s.c.* injection of two Orencia^{®} batches.
Fig. 8: Sialic Acid profile after de-O-acetylation (sample A sham treated control, sample E de-O-acetylated Orencia^{®}).
Fig. 9: Time course of mean serum levels (n=11-14/group) upon single *s.c.* injection of sham-modified or de-O-acetylated Orencia^{®} in rabbits.
Fig. 10: individual AUCs and group mean (n=11/group, bar indicates group mean) upon single *s.c.* injection of sham-modified or de-O-acetylated Orencia^{®} in rabbits.
Fig. 11: Relationship between mean AUC and level of O-acetylation (n=11-14/group) upon single *s.c.* injection of Orencia^{®} (sham-modified and de-O-acetylated material) in rabbits.
Fig. 12: individual Cₘₐₓ and group mean (n=11-14/group, bar indicates group mean) upon single *s.c.* injection of sham-modified or de-O-acetylated Orencia^{®} in rabbits.
Fig. 13: Relationship between mean Cₘₐₓ and level of O-acetylation (n=11-14/group) upon single *s.c.* injection of Orencia^{®} (sham-modified and de-O-acetylated material) in rabbits.
Fig. 14: Sialic Acid profile after de-O-acetylation (sample 1 de-O-acetylated Aranesp^{®}, sample 2 untreated control).
Fig. 15: Time course of Hb (normalized to Hb levels at baseline) following treatment with Aranesp^{®} and modified Aranesp^{®} (mean +SD, n=10/group).
Fig. 16: Time course of MCH following treatment with Aranesp^{®} and modified Aranesp^{®} (mean +SD, n=10/group).
Fig. 17: Some examples of N- or O-acetylated sialic acids.

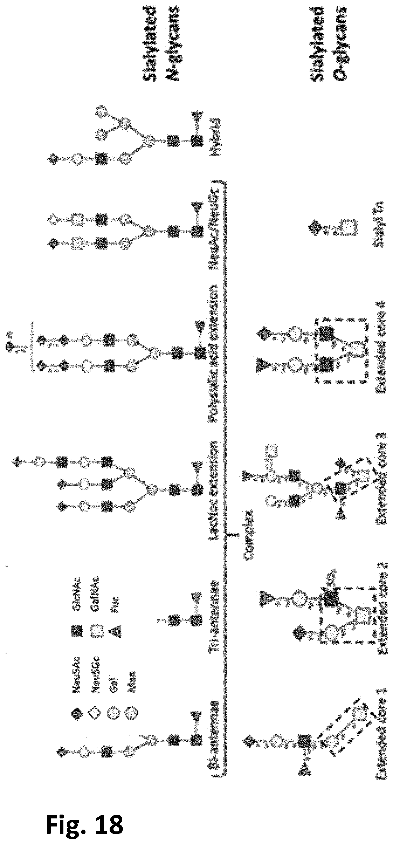

Fig. 18: General structure of N-linked glycans and O-linked glycans comprising acetylated sialic acids. N-linked glycans are attached to the protein in the endoplasmic reticulum to Asn in the sequence motif (Asn-X-Ser or Asn-X-Thr, where X is any AA acid except Pro). O-linked glycans are assembled one sugar at a time on a Ser or Thr residue in the Golgi apparatus. There seems to be no consensus motif, but presence of a Pro at either -1 or +3 relative to the Ser or Thr is favorable.

### Example 1: Effect of complete removal of glyco-structures on pharmacokinetics of a glycoprotein biologic

In a first study, the effect of complete removal of glyco-structures on the pharmacokinetics of a glycoprotein biologic was investigated. For this experiment, a glycosylated IgG1-type monoclonal antibody was used. While there is some evidence that galactosylation of the Fc domain of antibodies plays a role for the efficient recruitment of effector cells, the role of glycosylation as such, and in particular sialylation and O-acetylation of sialic acids, for the remaining characteristics such as pharmacokinetics, is less understood.

For this purpose, a human IgG1 mAb was enzymatically deglycosylated according to standard protocols (see, *e.g.,* Kim & Leahy 2013), and the pharmacokinetics were assessed following a single *i.v*. infusion, as summarized in Table 1.

**Table 1: Study Design, Single Dose PK Study in rabbits**

| **No.** | **Treatment** | **Dose** [mg/kg] | **volume** [mL/kg] | **schedule** | **route** | **N (f)** |
|---|---|---|---|---|---|---|
| 1 | Human IgG1 | 1.5 | 1.36 | single bolus infusion (t=0) | *i.v.* | 8 |
| 2 | Enzymatically deglycosylated human IgG1 | 1.5 | 1.5 | single bolus injection (t=0) | *i.v.* | 10 |

Dense serum samples were taken to closely monitor the entire time course up to 14 days following treatment, stored frozen and quantified for the human IgG concentrations using conventional ELISA, which was validated for this purpose.

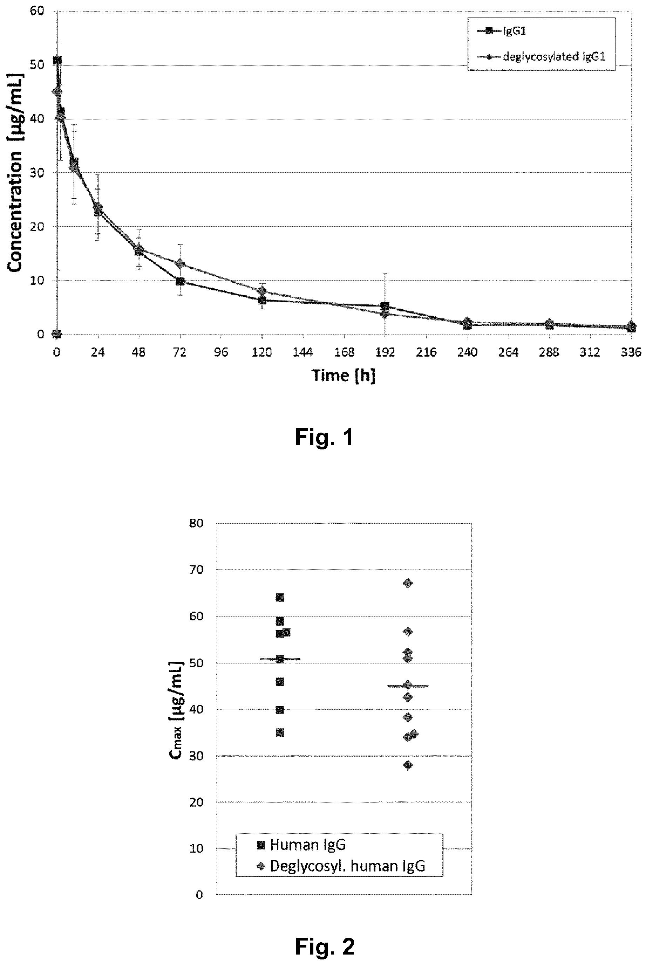

Fig. 1 indicates a fairly similar time course comparing the unmodified and de deglycosylated IgG, except the initial phase. Closer examining the initial phase, *i.e.,* Cₘₐₓ as illustrated in Fig. 2, the data show that although most glycostructures are not located on the outer surface of the protein backbone, these structures can well play a role not only on recruiting effector cells, but also distribution. Specifically, the results of this example show that complete removal of all glycans lowers maximal concentration.

### Example 2: Differences in bioavailability of different batches of a glycoprotein biologic with different sialic acid O-acetylation rates

It was tested whether two batches of the CTLA-FC fusion protein abatacept (Orencia^{®}) which have different levels of O-acetylation would differ in their exposure/bioavailability upon single, *s.c.* administration. The respective di-O-acetylation rates were 11.4% for batch No 1 and 6.5% for batch No 2. Tri-O-acetylated sialic acids were not observed.

**Table 2: Study Design, Single Dose PK Study in rabbits**

| **No.** | **Treatment** | **Dose [mg/kg]** | **volume [mL/kg]** | **schedule** | **route** | **N (f)** |
|---|---|---|---|---|---|---|
| 1 | Orencia^{®} Batch 1 | 5 | 0.62 | single injection (t=0) | *s.c.* | 11 |
| 2 | Orencia^{®} Batch 2 | 5 | 0.62 | single injection (t=0) | *s.c.* | 11 |

Dense serum samples were taken up to 14 days following treatment, to allow a close monitoring of serum levels, stored frozen and quantified for abatacept concentrations using conventional ELISA.

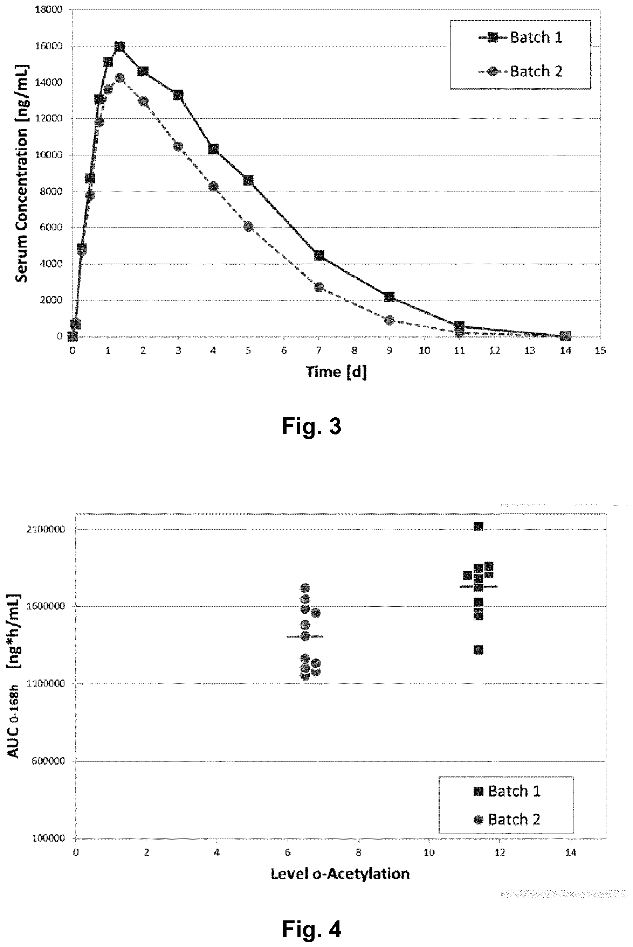

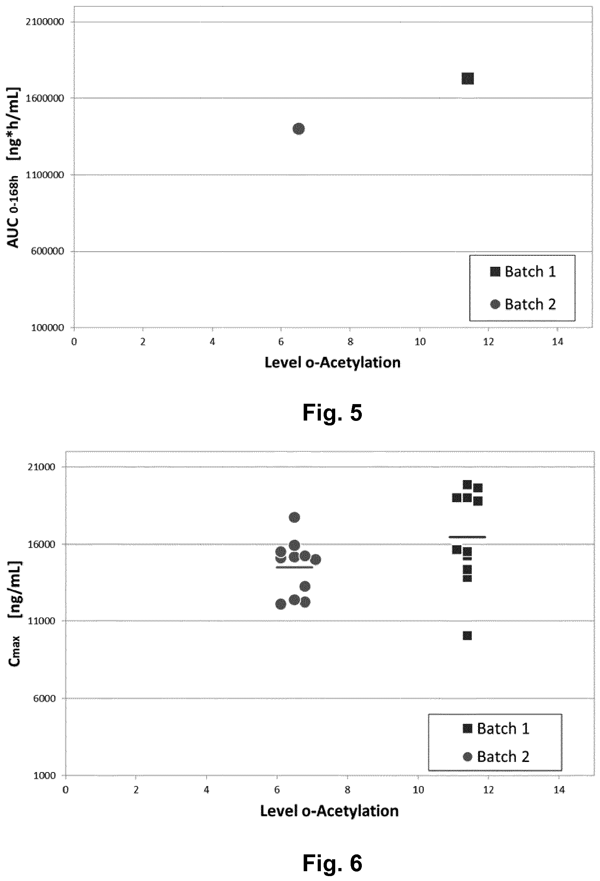

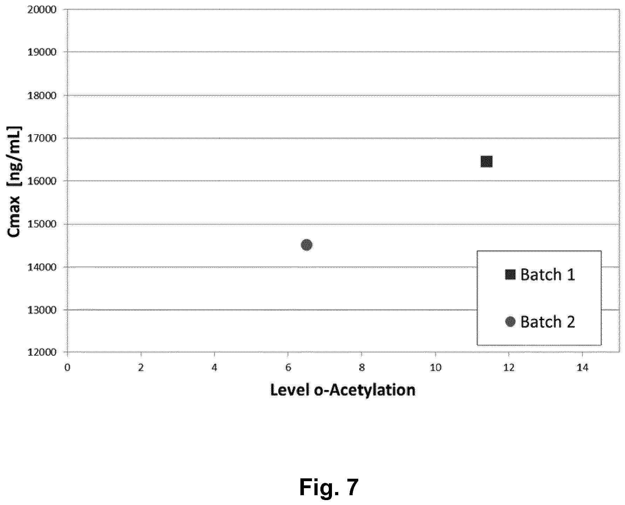

Fig. 3 shows the time course of mean serum levels (n=11/group) upon single *s.c.* injection of two Orencia^{®} batches in rabbits (reconstitution and further handling steps according to manufacturer instructions, applied slightly diluted in Orencia^{®} buffer to result in identical injection volume for both groups). Fig. 4 shows individual AUCs. Fig. 5 shows the relationship between mean AUC and level of O-acetylation. Fig. 6 shows individual AUC upon single *s.c.* injection of two Orencia^{®} batches in rabbits, and Fig. 7 shows the relationship between mean Cₘₐₓ and level of O-acetylation upon single *s.c.* injection of two Orencia^{®} batches.

Fig. 3 illustrates the time course of the mean serum concentration of the two batches used. Obviously, the two batches differ regarding the serum concentration achieved, although the same dose was administered. Fig. 4 further shows the individual AUCs observed for the animals in both groups and their different exposure. Fig. 5 illustrates the apparent relationship between level of O-acetylation and exposure. The difference is not only observed with regard to AUC, Fig. 6 illustrates that also the maximal serum concentration differs, like with AUC, apparently also correlating with Cₘₐₓ (Fig. 6 and 7). Hence, higher O-acetylation rates apparently increase bioavailability. This would be in line with the higher Cₘₐₓ observed for the IgG in example 1, as compared to a de-glycosylated and hence de-sialylated/de-O-acetylated IgG.

### Example 3: Effect of reduced sialic acid O-acetylation rates on exposure/bioavailability

In this example the causal relationship between O-acetylation rates and exposure/bioavailability of a selected glycoprotein biologic are investigated.

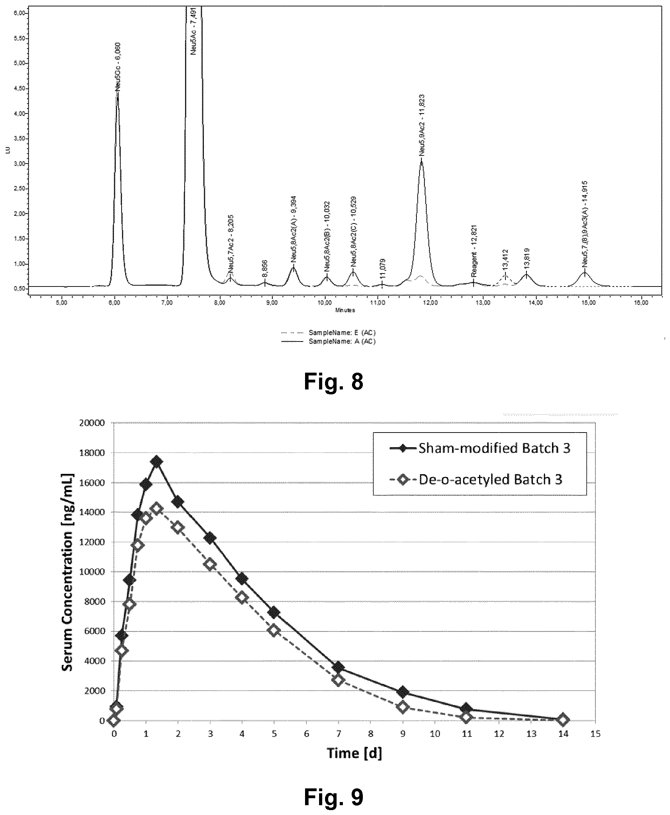

To this end, a sufficient amount of a single batch of abatacept (Orencia^{®}) was purchased, reconstituted and desalted into a 10 mM Sodium Phosphate / 1 mM MgCh buffer at pH 7. The batch was split into two halves. The first half was incubated with sialate-9-O-acetylesterase (Applied BioTech, Angewandte Biotechnologie GmbH) for two hours at 37°C. The second half was treated the same way, yet no enzyme was added. Subsequently the esterase was removed by affinity chromatopraphy. The characteristics of the two resulting materials is summarized in Table 3 and Fig 8. Table 4 summarizes the study design for the comparison the these two materials.

**Table 3: Characterization of de-O-acetylated and sham-treated Orencia^{®}:**

| **Method** | **Sham-treated Orencia^{®}** | **De-O-acetylated Orencia** |
|---|---|---|
| Potency (reporter gene assay) | 104% | 99% |
| Sialic acid profile by HPLC and DMB labelling* | % O-acetylation: 7.7 % | % O-acetylation: 2.3 % |
| Sialic acid content by IEC** | 9.6 mol SA / mol GPA2017 | 9.4 mol SA / mol GPA2017 |
| SEC purity | 98.2% | 98.4% |
| Glycan map | bG0: 9.1; bG1: 20.2, bG2: 53.6; tG3: 6.7; qG4: 2.1 | bG0: 9.5; bG1: 20.4, bG2: 53.4; tG3: 6.7; qG4: 2.1 |

| | | |
|---|---|---|
| *HPLC with sialic acids tagged with the fluorescent compound 1,2-diamino-4,5-methylene-dioxybenzene (DMB); ** IEC: cation exchange chromatography; ***SEC: size exclusion chromatography | | |

**Table 4: Study Design, Single Dose PK Study in rabbits**

| **No.** | **Treatment** | **Dose** [mg/kg] | **volume** [mL/kg] | **schedule** | **route** | **N (f)** |
|---|---|---|---|---|---|---|
| 1 | Sham modified Orencia^{®} Batch 3 | 5 | 0.62 | single injection (t=0) | *s.c.* | 14 |
| 2 | De-o-acetylated Orencia^{®} Batch 3 | 5 | 0.62 | single injection (t=0) | *s.c.* | 11 |

Dense serum sampling (at high frequency of sampling allowing a close and reliable monitoring of serum levels over time) was employed up to 14 days following treatment, with samples stored frozen and quantified for Orencia^{®} concentrations using conventional ELISA.

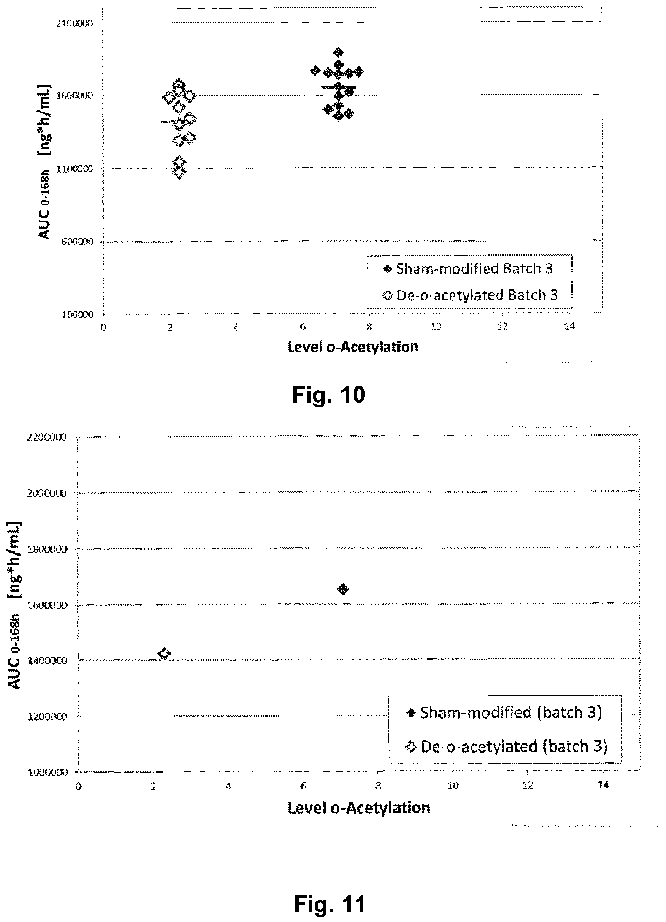

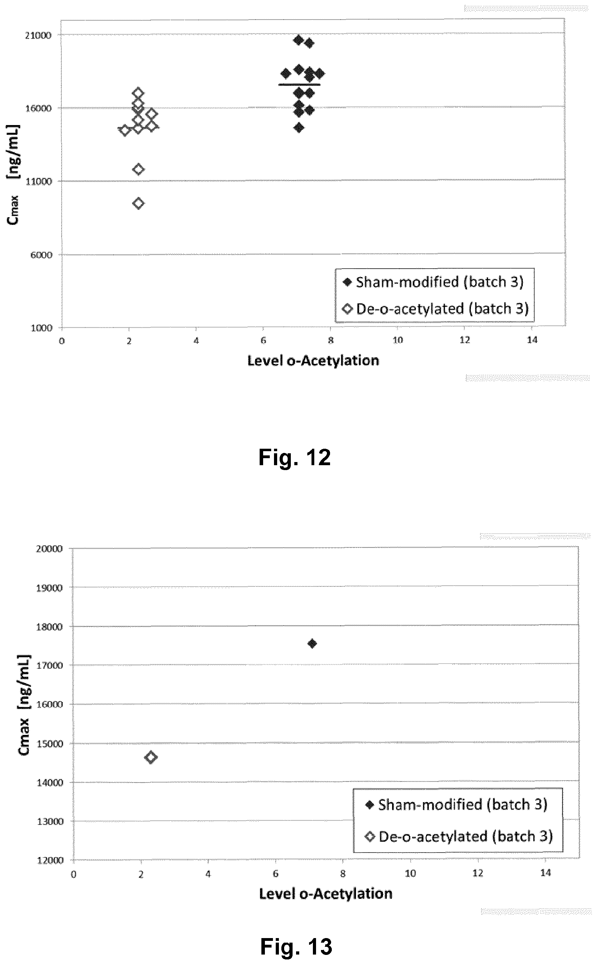

Doses of the two batches were then administered *s.c.* to rabbits. Results are shown in Figs 9 - 13. The time course of mean serum concentration (Fig. 9), the corresponding individual AUCs (Fig. 10, Fig. 11) clearly show that decreased O-acetylation rates result in a decrease of bioavailability. Cₘₐₓ was also lower for the material having decreased the lower O-acetylation rate (Fig. 12, Fig. 13).

### Example 4: Increased efficacy of an ESA with reduced level O-acetylated sialic acids

As typical example of erythropoiesis stimulating agents, Aranesp^{®} (darbepoetin alfa) was selected. Aranesp^{®} is highly sialylated and carries O-acetylated sialic acids as well. Rats were chosen as model due to the excellent predictivity of the results obtained for humans. As injection route, subcutaneous injection was chosen again, representing a typical route for clinical praxis. The dose range was selected to follow clinical praxis, as well.

For the preparation of de-O-acetylated Aranesp^{®} to be tested *in vivo* Aranesp^{®} was used. In short, several syringes were pooled to provide about 1 mg darpepoetin starting material for which the buffer was exchanged by dialysis into 50 mM Na-phosphate buffer pH 7.6 containing 140 mM NaCl, before incubation with 1 ml = which was treated with 1 U of sialate 9-O-acetylesterase for 20 h at 37°C.

After incubation the enzyme was removed by affinity chromatography on an anti-Epo antibody column. The column eluate was again buffer exchanged against 20 mM Na-phosphate buffer pH 6.2, containing 140 mM NaCl, filter-sterilized, aliquoted and stored below -60°C until used for the treatment of the animals as shown in this example. Content determination was done by RP-HPLC. The concentration measured by RP-HPLC was 0.136 mg/ml, which was used as basis to calculate the dosing.

The de-O-acetylated Aranesp^{®} was analyzed by LC-MS to determine the efficiency of the enzymatic treatment and control for the intact protein structure. Species with O-acetylated sialic acids were not detected, indicating efficient de-O-acetylation. Otherwise the spectrum show the expected glycosylated species with high abundance of molecules carrying tetra-antennary, tetra-sialo glycan structures at all five N-linked sites and one disialylated O-linked glycan (species with 22 sialic acids) and in general a high degree of sialylation. The distribution of glycoforms is also in qualitative agreement with the results of the sialo glycan maps.

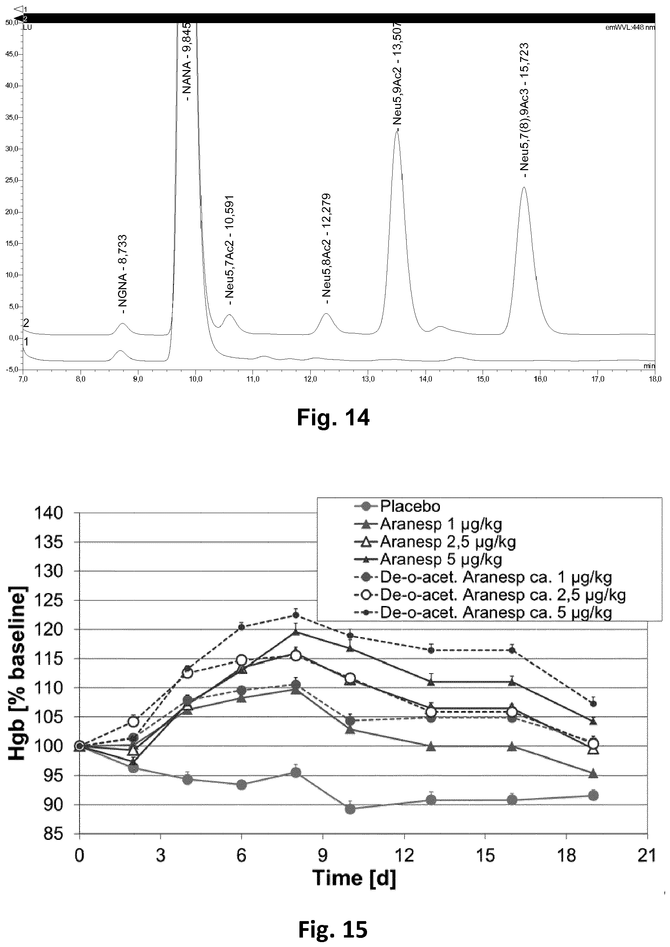

The analysis of sialic acids was conducted by DMB-labeling and RP-HPLC. The resulting chromatogram is shown in Fig. 14 in comparison with an untreated Aranesp^{®} sample. This analysis shows that the de-O-acetylation was very efficient, since only extremely low intensity peaks corresponding to mono- or di-O-acetylated sialic acids could be detected. The de-O-acetylated material was further characterized with respect to aggregation, also upon freeze-thaw cycles, and glycan analysis (sialo glycan maps by ion exchange chromatography). The characteristics of the two resulting materials are summarized in Table 5.

**Table 5: Characterization of de-O-acetylated and untreated Aranesp^{®}**

| **Method** | **Untreated Aranesp** | **De-O-acetylated Aranesp** |
|---|---|---|
| Sialic acid profile* | O-acetylation: 29.5% | O-acetylation: <1 %; |
| Glycan map** | Disialo: 1.3%; trisialo: 13.2%; tetrasialo: 85.4% | Disialo: 1.2%; trisialo: 13.0%; tetrasialo: 85.7% |
| SEC*** aggregation | n.d. | 0.05% |

| | | |
|---|---|---|
| n.d.: not detected, *RP-HPLC of sialic acids tagged with the fluorescent compound 1,2-diamino-4,5-methylenedioxybenzene (DMB); ^{∗∗}Cation exchange chromatography of glycans tagged with the 2-aminobenzamide (2AB); ***SEC: size exclusion chromatography. | | |

Rats were chosen as species for the comparison due to the excellent predictivity of the results obtained for humans. As injection route, subcutaneous injection was chosen, representing a typical route for clinical praxis. The dose range was selected to follow clinical praxis, as well.

Due to the sensitivity of PD read-outs, the study design was further refined in this example. In addition to the highly specific enzymatic modification, the dosing considered the change of the molecular weight by the modification. The molecular weight of native Aranesp^{®} is about 37, 100 Da. In case of a complete de-O-acetylation, the MW decreases to about 36,366 Da. While the dose was weight based (mg/kg) in the previous examples, the dose was the same in this study based on the number of molecules administered, *i.e*., equimolar dosing, considering the 1-2 % change in molecular weight (Table 6).

**Table 6: Study Design, Single Dose PK Study in rats**

| **No.** | **Treatment** | **Dose** [mol/kg] | **Dose** [µg/kg] | **volume** [mL/kg] | **Schedule** | **route** | **N (m/f)** |
|---|---|---|---|---|---|---|---|
| 1 | Placebo | - | - | 1.0 | single injection (t=0) | *s.c.* | 0/10 |
| 2 | Aranesp^{®} | 2.70e-11 | 1 | 1.0 | single injection (t=0) | *s.c.* | 0/10 |
| 3 | Aranesp^{®} | 6.74e-11 | 2.5 | 1.0 | single injection (t=0) | *s.c.* | 0/10 |
| 4 | Aranesp^{®} | 13.5e-11 | 5 | 1.0 | single injection (t=0) | *s.c.* | 0/10 |
| 5 | Low O-acetyl Aranesp^{®} | 2.70e-11 | ca. 1 | 1.0 | single injection (t=0) | *s.c.* | 0/10 |
| 6 | Low O-acetyl Aranesp^{®} | 6.74e-11 | ca. 2.5 | 1.0 | single injection (t=0) | *s.c.* | 0/10 |
| 7 | Low O-acetyl Aranesp^{®} | 13,5e-11 | ca. 5 | 1.0 | single injection (t=0) | *s.c.* | 0/10 |

The animals were housed under standard conditions and treated with a single injection as detailed in Table 6. The assignment to the treatment groups was performed randomly, prior to dosing. Blood was sampled from the tail vein and analyzed using standard hematological equipment.

Table 7 summarizes the minimal level of O-acetylation observed for a substantial number of Aranesp^{®} lots, illustrating that O-acetylation of Neu5Ac consistently plays a major role with regard to the overall mean level of O-acetylation.

**Table 7: Minimal level of O-acetylation observed for Aranesp^{®}**

| **Position** | **Minimal level** |
|---|---|
| Neu5Gc (NGNA) | 0.6% |
| Neu5Ac (NANA) | 67.9% |
| Neu5Ac-O-acetylated | 27.2% |
| Neu5Gc-O-acetylated | n.d. |

In order to compensate for minimal baseline differences of Hb between groups, *i.e.,* prior to treatment, Fig. 15 illustrates Hb observed as % of baseline levels. The data demonstrate a slightly more pronounced increase of Hb following treatment with modified Aranesp^{®} as compared to native Aranesp^{®}. The more pronounced increase was consistently observed at all three dose levels tested.

To gain more insight into the details of Hb increase, reticulocyte as well as erythrocyte count was examined. Unexpectedly, the level of RBC increase in groups treated with modified Aranesp^{®} was slightly less pronounced than observed in the Aranesp^{®} treated groups.

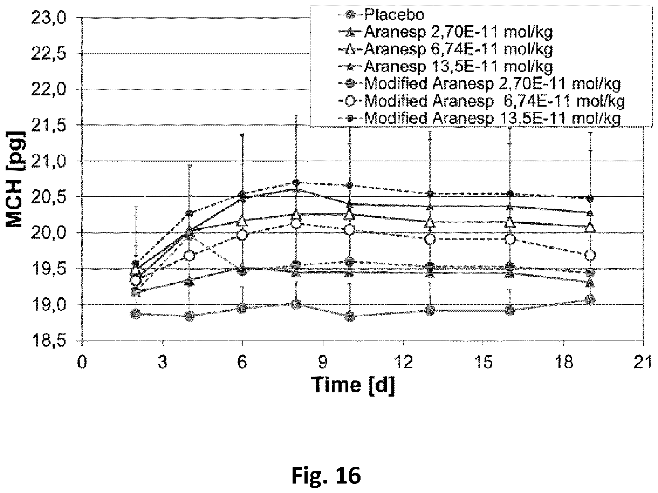

Consequently, the MCH was analyzed as illustrated in Fig. 16. Surprisingly, synchronous with the increased Hb observed following treatment, the MCH increase following treatment with the modified Aranesp^{®} was more pronounced than for native Aranesp^{®}.

As a result, the ESA with de- or non-acetylated sialic acids showed less pronounced stimulation of RBC proliferation, but an increased MCH in the resulting RBCs, compared to unmodified ESA.

Because RBCs participate in hemostasis through exposure of procoagulant phospholipids (Peyrou *et al.,* 1999), an ESA-mediated increase of RBCs results in an increased cardiovascular risk. However, the therapeutic goal of ESA treatment is the increase of oxygen capacity of the blood, not necessarily the increase of RBCs. Conventional ESA treatment accomplishes this by stimulation of RBC proliferation, hence resulting in a higher oxygen capacity.

The inventors have shown that ESA with de- or non-acetylated sialic acids still increase oxygen capacity of the blood, however, without increasing the RBC to the same extent as conventional ESA, but by increasing the MCH, *i.e.,* the average load of Hb per RBC. This approach may help to reduce side effects that coincide with ESA treatment, like an increased cardiovascular risk.

Without being bound to theory, one explanation may be that the amount of Hb loaded into RBC during maturation can differ. While some conditions characterized by hypochromic anemias demonstrate normal RBC counts but low Hb, because there is a disproportionate reduction of Hb relative to the volume of the RBC, it appears that ESA with de- or non-acetylated sialic cause the opposite phenomenon appears, in that the packing of Hb/RBC is increased. Generally, sialic acids are examples for glycostructures that are known to play an important role in numerous biological processes. Genetic modification of therapeutic proteins to increase the level of sialylation is a successful and frequently used approach to maximize exposure resulting from an administered dose. It is surprising that a decreased level of O-acetylation of sialic acids in an ESA consistently leads, on one hand, to a lower level of exposure/bioavailability, but at the same time to an increased efficacy in terms of MCH. This is unexpected, because publications on erythropoetin show that O-acetylation of sialic acids actually increases the half-life (Shahrokh *et al.,* 2011).

Accordingly, one would have expected that ESA with lower level of O-acetylation of sialic acids would have a lower efficacy in terms of oxygen capacity, but the observed effect was the opposite.

### References

Reuter & Schauer (1994), Methods in enzymology vol 230, p. 168-199
Kim & Leahy (2013), Methods in enzymology vol. 533 p. 259-63
Sojar & Bahl (1987), Arch Biochem Biophys. Nov 15; 259(1):52-7
Johnson, & Jolliffe (2000), Nephrol. Dial. Transplant. vol. 15 (9) p. 1274-1277
Middleton et al. (1999), J. Biol. Chem. vol. 274 (20) p. 14163-14169
Baumann et al. (2015), Nature communications vol. 6 p. 7673
Peyrou et al. (1999), Thromb Haemost. Vol 81(3): 400-406
Gaj et al. (2013), Trends in biotechnology vol. 31 (7) p. 397-405
Shahrokh et al. (2011) Molecular pharmaceutics vol. 8 (1) p. 286-96
Hua et al. (2015) Trends in analytical chemistry Vol. 68 p. 18-27
Reichel and Gmeiner (2009) Handbook of Experimental Pharmacology/Doping in Sports: Biochemical Principles, Effects and Analysis, Springer, p. 251-254

## Claims

1. A method or process of producing an erythropoiesis-stimulating agent (ESA) selected from the group consisting of erythropoietin, a modified erythropoietin or an erythropoietin mimetic, wherein the method or process comprises
the heterologous expression of said erythropoiesis-stimulating agent (ESA) in a suitable expression system,
wherein at least one step or feature is provided that results in a reduced O-acetylation rate of sialic acid residues in said erythropoiesis-stimulating agent (ESA),
for the purpose of increasing mean corpuscular hemoglobin (MCH).

2. The method or process of claim 1, wherein the step or feature that results in a reduced O-acetylation rate of sialic acid residues is at least one selected from the group consisting of:
a) O-deacetylation of sialic acid residues in glycans of said erythropoiesis-stimulating agent (ESA),
b) reduction of overall glycosylation of said erythropoiesis-stimulating agent (ESA), resulting in a reduction of O-acetylated sialic acid residues,
c) use of an expressor cell line that is capable of expressing erythropoiesis-stimulating agent (ESA) that has de- or non-O-acetylated sialic acid residues, or a reduced O-acetylation rate of sialic acid residues
d) use of an expressor cell line that is capable of expressing erythropoiesis-stimulating agent (ESA) that is deglycosylated, or have reduced glycosylation
e) reduction of sialic acid content in glycans of said erythropoiesis-stimulating agent (ESA), and
f) use of an expressor cell line that is capable of expressing erythropoiesis-stimulating agent (ESA) that has a reduced amount of sialic acid, or lack sialic acids.

3. The method or process according to any of the aforementioned claims, wherein the erythropoiesis-stimulating agent (ESA) is at least one selected from the group consisting of:
• Epoetin α (Epogen^{®}, ESPO^{®}, Procrit^{®}, Eprex^{®}, Erypo^{®}, Epoxitin^{®}, Globuren^{®}, Epopen^{®}, Epoglobin^{®}, Epox^{®}, Eritrogen^{®})
• Epoetin-β (NeoRecormon^{®}, Epogin^{®})
• Darbepoetin α (Aranesp^{®}, Nespo^{®})
• CERA (Continuous Erythropoiesis Receptor Activator)
• ErepoXen^{®}
• Albupoetin^{®}
• PT-401
• Epo-Fc
• CEPO (carbamylated EPO)
• MOD-7023
• Epoetin δ (DynEpo^{®})
• GO-EPO
• MK2578

4. The method or process according to any of the aforementioned claims, wherein said erythropoiesis-stimulating agent (ESA) has at least one selected from the group consisting of:
a) an absolute acetylation rate of ≤ 10 %, and
b) an acetylation rate that is reduced by ≥ 50 %.

5. *In-vitro* use of an erythropoiesis-stimulating agent (ESA), selected from the group consisting of erythropoietin, a modified erythropoietin or an erythropoietin mimetic, comprising a reduced O-acetylation rate of sialic acid residues for the increase of mean corpuscular hemoglobin (MCH).

6. An erythropoiesis-stimulating agent (ESA), selected from the group consisting of erythropoietin, a modified erythropoietin or an erythropoietin mimetic, comprising a reduced O-acetylation rate of sialic acid residues, for use in the treatment of conditions associated with a reduced mean corpuscular hemoglobin (MCH).

7. The ESA according to claim 6, wherein said condition is a condition associated with normal red blood cell (RBC) counts but low hemoglobin (Hb).

8. The ESA according to any one of claims 6 or 7, wherein said condition is hypochromic anemia.

## Patentansprüche

1. Ein Verfahren oder Prozess zur Herstellung eines Erythropoese-stimulierenden Agens (ESA), ausgewählt aus der Gruppe bestehend aus Erythropoetin, einem modifizierten Erythropoetin, oder einem Erythropoetin-Mimetikum, wobei das Verfahren oder der Prozess die heterologe Expression von besagtem Erythropoese-stimulierenden Agens (ESA) in einem geeigneten Expressionssystem umfasst,
wobei mindestens ein Schritt oder Merkmal bereitgestellt wird, der in einer reduzierten O-Azetylierungsrate von Sialinsäureresten in besagtem Erythropoese-stimulierenden Agens (ESA) resultiert,
zum Zwecke der Erhöhung des mittleren korpuskulären Hämoglobins (MCH).

2. Das Verfahren oder der Prozess gemäß Anspruch 1, wobei der Schritt oder das Merkmal, der in einer reduzierten O-Azetylierungsrate von Sialinsäureresten resultiert, mindestens einer ist ausgewählt aus der Gruppe bestehend aus:
a) O-Deazetylierung von Sialinsäureresten in Glykanen von besagtem Erythropoesestimulierendem Agens (ESA),
b) Reduktion der gesamten Glykosylierung von besagtem Erythropoesestimulierendem Agens (ESA), welche in einer Reduktion von O-azetylierten Sialinsäureresten resultiert,
c) Verwendung einer Expressionszelllinie, die zur Expression eines Erythropoese-stimulierenden Agens (ESA) befähigt ist, das de- oder nicht-O-azetylierte Sialinsäurereste oder eine reduzierte O-Azetylierungsrate von Sialinsäureresten aufweist,
d) Verwendung einer Expressionszelllinie, die zur Expression eines Erythropoese-stimulierenden Agens (ESA) befähigt ist, das de-glykosyliert ist oder eine reduzierte Glykosylierung aufweist,
e) Reduktion des Sialinsäuregehalts in den Glykanen des besagten Erythropoese-stimulierenden Agens (ESA), und
f) Verwendung einer Expressionszelllinie, die zur Expression eines Erythropoese-stimulierenden Agens (ESA) befähigt ist, das eine reduzierte Menge von Sialinsäure aufweist, oder gar keine Sialinsäuren.

3. Das Verfahren oder der Prozess gemäß einem der vorgenannten Ansprüche, wobei das Erythropoese-stimulierende Agens (ESA) mindestens eines ist, das ausgewählt ist aus der Gruppe bestehend aus:
• Epoetin α (Epogen^{®}, ESPO^{®}, Procrit^{®}, Eprex^{®}, Erypo^{®}, Epoxitin^{®}, Globuren^{®}, Epopen^{®}, Epoglobin^{®}, Epox^{®}, Eritrogen^{®})
• Epoetin-β (NeoRecormon^{®}, Epogin^{®})
• Darbepoetin α (Aranesp^{®}, Nespo^{®} )
• CERA (Continuous Erythropoiesis Receptor Activator)
• ErepoXen^{®}
• Albupoetin^{®}
• PT-401
• Epo-Fc
• CEPO (carbamyliertes EPO)
• MOD-7023
• Epoetin δ (DynEpo^{®})
• GO-EPO
• MK2578

4. Das Verfahren oder der Prozess gemäß einem der vorgenannten Ansprüche, wobei besagtes Erythropoese-stimulierendes Agens (ESA) mindestens eines aufweist aus der Gruppe bestehend aus:
a) einer absoluten Azetylierungsrate von ≤ 10 %, und
b) einer Azetylierungsrate, die um ≥ 50 % reduziert ist.

5. Verwendung eines Erythropoese-stimulierenden Agens (ESA) *in-vitro,* ausgewählt aus der Gruppe bestehend aus Erythropoetin, einem modifizierten Erythropoetin, oder einem Erythropoetin-Mimetikum, das eine reduzierte O-Azetylierungsrate von Sialinsäureresten aufweist zum Zwecke der Erhöhung des mittleren korpuskulären Hämoglobins (MCH).

6. Ein Erythropoese-stimulierenden Agens (ESA), ausgewählt aus der Gruppe bestehend aus Erythropoetin, einem modifizierten Erythropoetin, oder einem Erythropoetin-Mimetikum, umfassend eine reduzierte O-Azetylierungsrate von Sialinsäureresten, zur Verwendung bei der Behandlung von Zuständen, die mit einem reduzierten mittleren korpuskulären Hämoglobin (MCH) einhergehen.

7. Das ESA gemäß Anspruch 6, wobei besagter Zustand ein Zustand ist, der mit einer normalen Anzahl von roten Blutkörperchen, aber einem niedrigen Hämoglobin (Hb) einhergeht.

8. Das ESA gemäß einem der Ansprüche 6 oder 7, wobei besagter Zustand eine hypochrome Anämie ist.

## Revendications

1. Procédé ou processus de production d'un agent de stimulation de l'érythropoïèse (ESA) sélectionné dans le groupe constitué par l'érythropoïétine, une érythropoïétine modifiée ou un mimétique d'érythropoïétine, dans lequel le procédé ou processus comprend
l'expression hétérologue dudit agent de stimulation de l'érythropoïèse (ESA) dans un système d'expression approprié,
dans lequel au moins une étape ou fonction est prévue qui résulte en un taux de O-acétylation réduit de résidus d'acide sialique dans ledit agent de stimulation de l'érythropoïèse (ESA),
dans le but d'augmenter l'hémoglobine corpusculaire moyenne (MCH).

2. Procédé ou processus selon la revendication 1, dans lequel l'étape ou fonction qui aboutit à un taux de O-acétylation réduit de résidus d'acide sialique est au moins une étape sélectionnée dans le groupe constitué par :
a) la O-désacétylation de résidus d'acide sialique dans les glycanes dudit agent de stimulation de l'érythropoïèse (ESA),
b) la réduction de glycosylation complète dudit agent de stimulation de l'érythropoïèse (ESA), aboutissant à une réduction de résidus d'acide sialique O-acétylés,
c) l'utilisation d'une lignée cellulaire d'agent d'expression qui est susceptible d'exprimer l'agent de stimulation de l'érythropoïèse (ESA) qui comporte des résidus d'acide sialique dé- ou non O-acétylés, ou un taux de O-acétylation réduit de résidus d'acide sialique ,
d) l'utilisation d'une lignée cellulaire d'agent d'expression qui est susceptible d'exprimer l'agent de stimulation de l'érythropoïèse (ESA) qui est déglycosylé, ou le fait d'avoir une glycosylation réduite,
e) la réduction de la teneur en acide sialique dans les glycanes dudit agent de stimulation de l'érythropoïèse (ESA), et
f) l'utilisation d'une lignée cellulaire d'agent d'expression qui est susceptible d'exprimer l'agent de stimulation de l'érythropoïèse (ESA) qui a une quantité réduite d'acide sialique, ou un manque d'acides sialiques.

3. Procédé ou processus selon l'une quelconque des revendications précédentes, dans lequel l'agent de stimulation de l'érythropoïèse (ESA) est au moins un agent sélectionné dans le groupe constitué par :
• Époétine-α (Epogen^{®}, ESPO^{®}, Procrit^{®}, Eprex^{®}, Erypo^{®}, Epoxitin^{®}, Globuren^{®}, Epopen^{®}, Epoglobin^{®}, Epox^{®}, Eritrogen^{®})
• Époétine-β (NeoRecormon^{®}, Epogin^{®})
• Darbépoétine-α (Aranesp^{®}, Nespo^{®})
• CERA (Activateur Continu du Récepteur à l'Érythropoïétine)
• ErepoXen^{®}
• Albupoetin^{®}
• PT-401
• Epo-Fc
• CEPO (EPO carbamylé)
• MOD-7023
• Époétine-δ (DynEpo^{®})
• GO-EPO
• MK2578

4. Procédé ou processus selon l'une quelconque des revendications précédentes, dans lequel ledit agent de stimulation de l'érythropoïèse (ESA) a au moins un taux sélectionné dans le groupe constitué par :
a) un taux d'acétylation absolu ≤ 10 %, et
b) un taux d'acétylation qui est réduit de ≥ 50 %.

5. Utilisation *in vitro* d'un agent de stimulation de l'érythropoïèse (ESA), sélectionné dans le groupe constitué par l'érythropoïétine, une érythropoïétine modifiée ou un mimétique d'érythropoïétine, comprenant un taux de O-acétylation réduit de résidus d'acide sialique pour l'augmentation de l'hémoglobine corpusculaire moyenne (MCH) .

6. Agent de stimulation de l'érythropoïèse (ESA), sélectionné dans le groupe constitué par l'érythropoïétine, une érythropoïétine modifiée ou un mimétique d'érythropoïétine, comprenant un taux de O-acétylation réduit de résidus d'acide sialique , pour une utilisation dans le traitement d'états associés à une hémoglobine corpusculaire moyenne réduite (MCH).

7. ESA selon la revendication 6, dans lequel ledit état est un état associé à des nombres normaux de globules rouges (RBC), mais à une hémoglobine basse (Hb).

8. ESA selon l'une quelconque des revendications 6 ou 7, dans lequel ledit état est une anémie hypochrome.
